(19) Europäisches Patentamt
European Patent Office
Office européen des brevets

(11) Veröffentlichungsnummer: **0 286 928**
**A2**

# (12) EUROPÄISCHE PATENTANMELDUNG

(21) Anmeldenummer: **88105332.6**

(22) Anmeldetag: **01.04.88**

(51) Int. Cl.[4]: **C07D 207/08 , C07D 405/12 , C07D 405/14 , C07K 5/06 , C07K 5/08 , A61K 31/40 , A61K 37/02**

(30) Priorität: **11.04.87 DE 3712365**

(43) Veröffentlichungstag der Anmeldung: **19.10.88 Patentblatt 88/42**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Henning, Rainer, Dr.**
**Rotenhofstrasse 31**
**D-6234 Hattersheim am Main(DE)**
Erfinder: **Hock, Franz, Dr.**
**Altstadt 19**
**D-6110 Dieburg(DE)**
Erfinder: **Urbach, Hansjörg, Dr.**
**Le Lavandoustrasse 41**
**D-6242 Kronberg/Taunus(DE)**

(54) **Neue 2-Acylpyrrolidin-Derivate, Verfahren zu ihrer Herstellung, sie enthaltende Mittel sowie deren Verwendung.**

(57) Die Erfindung betrifft Pyrrolidin-Derivate der allgemeinen Formel

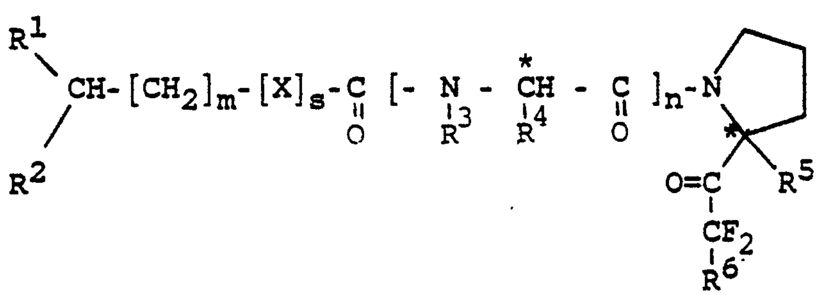

in welcher $R^1$, $R^2$, $R^3$, $R^4$, $R^5$ und $R^6$ die in der Beschreibung angegebenen Bedeutungen haben; X Sauerstoff, Imino oder N-Alkylimino bedeutet; m = 0-5 ist; n = 0-2 ist und s = 0 oder 1 ist; Verfahren zu ihrer Herstellung, sie enthaltende Mittel sowie deren Verwendung.

**EP 0 286 928 A2**

# Neue 2-Acylpyrrolidin-Derivate, Verfahren zu ihrer Herstellung, sie enthaltende Mittel sowie deren Verwendung

Die Erfindung betrifft Pyrrolidin-Derivate der allgemeinen Formel I

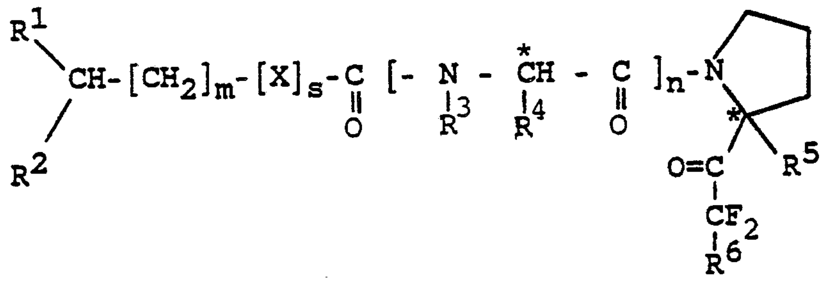

(I)

in welcher

$R^1$ Wasserstoff; $(C_1-C_6)$-Alkyl oder $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino oder ein ($C_1$ oder $C_2$)-Alkylendioxy substituiert ist, bedeutet;

$R^2$ Wasserstoff; $(C_1-C_6)$-Alkyl; $(C_6-C_{12})$-Aryl; $(C_6-C_{12})$-Aryloxy; $(C_7-C_{13})$-Aroyl; Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist;

oder

$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist;

$R^3$ Wasserstoff; $(C_1-C_6)$-Alkyl; $(C_6-C_{12})$-Aryl-$(C_1-C_6)$-alkyl; $(C_5-C_9)$-Cycloalkyl; Indanyl oder Tetrahydronaphthyl bedeutet;

$R^4$ Wasserstoff; $(C_1-C_6)$-Alkyl, das durch Amino, $(C_1-C_6)$-Acylamino, insbesondere $(C_1-C_6)$-Alkanoylamino oder Boc-NH, oder Benzoylamino monosubstituiert sein kann; $(C_2-C_6)$-Alkenyl; $(C_5-C_9)$-Cycloalkyl; $(C_5-C_9)$-Cycloalkenyl; $(C_5-C_7)$-Cycloalkyl-$(C_1-C_4)$-alkyl; $(C_6-C_{12})$-Aryl oder teilhydriertes $(C_6-C_{12})$-Aryl, die jeweils durch einen, zwei oder drei gleiche oder verschiedene Reste aus der Reihe $(C_1-C_4)$-Alkyl, ($C_1$ oder $C_2$)-Alkoxy und Halogen substituiert sein können; $(C_6-C_{12})$-Aryl-$(C_1-C_4)$-alkyl oder $(C_7-C_{13})$-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen; oder, falls von vorstehenden Definitionen noch nicht umfaßt, die gegebenenfalls geschützte Seitenkette einer natürlich vorkommenden α-Aminosäure bedeutet, oder $R^3$ und $R^4$ zusammen für $-[CH_2]_p-$ stehen, worin p = 3, 4 oder 5 ist und worin eine Methylengruppe durch S oder O ersetzt werden kann;

$R^5$ Wasserstoff; $(C_1-C_6)$-Alkyl; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^6$ $(C_1-C_8)$-Alkyl; $(C_4-C_{10})$-Cycloalkyl oder $(C_4-C_{10})$-Cycloalkyl-$(C_1-C_4)$-alkyl, wobei ein Cycloalkylrest auch, abhängig von der Ringgröße, bis zu 4 Doppelbindungen enthalten und/oder bis zu dreifach verbrückt sein kann; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist; Fluor oder einen Rest $C_qH_{(2q+1-r)}F_r$ mit q = 1, 2, 3, 4 oder 5 und r = eine ganze Zahl von 1 bis (2q+1), bedeutet;

X Sauerstoff; Imino oder N-$(C_1-C_8)$-Alkylimino bedeutet;

m 0, 1, 2, 3, 4 oder 5 ist;

n 0, 1 oder 2 ist und

s 0 oder 1 ist;

sowie deren physiologisch verträgliche Salze, falls solche gebildet werden können.

Alkyl kann geradkettig oder verzweigt sein und ist beispielsweise Methyl, Ethyl, Propyl, Isopropyl, Butyl,

Isobutyl, sec.-Butyl, tert.-Butyl, Pentyl, Isopentyl, Neopentyl, sec.-Pentyl, tert.-Pentyl, Hexyl, Isohexyl, Heptyl oder Octyl. Entsprechendes gilt für davon abgeleitete Reste wie Alkoxy, Alkylamino, Dialkylamino, Alkanoyl, Alkoxycarbonyl und Aralkyl.

Aryl ist beispielsweise Phenyl, α-oder β-Naphthyl, 2-, 3-oder 4-Biphenylyl; bevorzugt ist Phenyl. Entsprechendes gilt für davon abgeleitete Reste wie Aryloxy, Aralkyl, Aryl, Aroyl und Arylalkanoyl.

Halogen ist Fluor, Chlor, Brom oder Jod; bevorzugt sind Fluor, Chlor und Brom.

Unter $R^4$ in der Bedeutung eines mono-bzw. bicyclischen Heterocylen-Restes mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen, wird beispielsweise Thienyl, Benzo[b]-thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Verbindungen der Formel I besitzen chirale C-Atome. Sowohl die R-als auch die S-Konfigurationen an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen die mit einem Stern (*) markierten C-Atome S-Konfiguration aufweisen. Falls $R^4$ für die Seitenkette von Cystein steht, wird jedoch die R-Konfiguration dieses Zentrums bevorzugt.

Natürlich vorkommende α-Aminosäuren, wie beispielsweise Ala, Val, Leu, Ile, Phe, Abu, C-Ph-Gly, His, Trp, Lys, Orn, Dab, Dap, Daap, Dapi, Arg, Cit, Glu, Gln, Asp, Asn, Cys, Met, Hyl, Ser, Thr und Tyr sind z.B. in Ann. Rev. Biochem. 38 [1969] 137-158 und FEBS Letters 64 [1976] 29-35 beschrieben.

Falls $R^4$ für eine geschützte Seitenkette einer natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. z.B. T.W. Greene, "Protective Groups in Organic Synthesis, New York, 1981 oder Bodanszky, Bodanszky, "Principles bzw. Practice of Peptide Synthesis", Berlin, 1984). Im Falle, daß $R^4$ eine geschützte Lysin-Seitenkette bedeutet, werden die bekannten Aminoschutzgruppen, insbesondere aber ($C_1$-$C_6$)-Alkanoyl bevorzugt. Falls $R^4$ eine geschützte Tyrosin-Seitenkette bedeutet, wird die Ether-Schutzgruppe am Sauerstoff, insbesondere ($C_1$-$C_6$)-Alkyl, bevorzugt; besonders bevorzugte Schutzgruppen sind Methyl und Ethyl.

Als Salze kommen insbesondere Alkali-oder Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Bevorzugt sind Verbindungen der Formel I, in welcher

$R^1$ Wasserstoff; ($C_1$-$C_4$)-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder ein Methylendioxy substituiert sind; oder

$R^1$ und $R^2$ zusammen für Benzyliden stehen, worin der Phenylrest gegebenenfalls durch einen oder zwei gleiche oder verschiedenen Reste aus der Reihe Methyl, Ethyl, Methoxy, Fluor, Chlor, Brom und Hydroxy, drei Methoxy, oder ein Methylendioxy substituiert ist;

$R^3$ Wasserstoff; ($C_1$-$C_4$)-Alkyl; Benzyl; Phenethyl; Cyclopentyl; Cyclohexyl oder Indanyl bedeutet;

$R^4$ Wasserstoff oder die gegebenenfalls geschützte Seitenkette einer natürlich vorkommenden α-Aminosäure bedeutet; oder

$R^3$ und $R^4$ zusammen für einen Rest -$[CH_2]_p$-stehen, der wie im Anspruch 1 definiert ist und worin p = 3 oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ ($C_1$-$C_8$)-Alkyl; ($C_5$-$C_8$)-Cycloalkyl; Phenyl; Benzyl; Phenethyl; Fluor oder Perfluor-($C_1$-$C_5$)-alkyl bedeutet, wobei Phenyl, Benzyl und Phenethyl im Phenylrest durch einen, zwei oder drei gleiche oder verschiedene Reste aus der Reihe Methyl, Ethyl, Methoxy, Ethoxy, Fluor, Chlor und Brom, oder ein Methylendioxy substituiert sein können;

X Sauerstoff bedeutet;

m = 0, 1, 2, 3, 4 oder 5 ist;

n = 0 oder 1 ist und
s = 0 oder 1 ist
sowie deren physiologisch verträgliche Salze.

Besonders bevorzugt sind Verbindungen der Formel I

$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 1,3-, 2,4-, 2,5-, 2,6-, 3,4-oder 3,5-Dimethoxyphenyl bedeutet;

$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder

$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-oder 3,5-Dimethoxybenzyliden stehen;

$R^3$ Wasserstoff, Methyl oder Benzyl bedeutet;

$R^4$ Wasserstoff, Methyl; n-Butyl; Isopropyl; Isobutyl; sec.-Butyl oder Benzyl bedeutet; oder

$R^3$ und $R^4$ zusammen für $-[CH_2]_3-$stehen;

$R^5$ Wasserstoff oder Methyl bedeutet;

$R^6$ $(C_1-C_6)$-Alkyl; $(C_5-C_8)$-Cycloalkyl; Phenyl; Benzyl; Phenethyl; Fluor oder Perfluor-$(C_1-C_5)$-alkyl bedeutet, wobei Phenyl, Benzyl und Phenethyl im Phenylrest durch einen oder zwei gleiche oder verschiedene Reste aus der Reihe Methyl, Methoxy, Fluor und Chlor, drei Methoxy, oder ein Methylendioxy substituiert sein können;

X Sauerstoff bedeutet;
m = 0, 1, 2, 3, 4 oder 5 ist;
n = 0 oder 1 ist und
s = 0 oder 1 ist
sowie deren physiologisch verträgliche Salze.

Insbesondere bevorzugt sind solche Verbindungen der Formel I, in welcher

$R^2$ Wasserstoff bedeutet;
$R^3$ und $R^4$ zusammen für $-[CH_2]_3-$stehen;
$R^5$ Wasserstoff bedeutet;
X Sauerstoff bedeutet;
m = 1, 2, 3, oder 4 ist;
n = 1 ist und
s = 0 oder 1 ist,
sowie solche Verbindungen der Formel I, in welcher
$R^5$ Wasserstoff bedeutet;
X Sauerstoff bedeutet;
m = 1, 2, 3 oder 4 ist;
n = 0 ist und
s = 0 oder 1 ist.

Die Erfindung betrifft auch ein Verfahren zur Herstellung von Verbindungen der Formel I, das dadurch gekennzeichnet ist, daß man

a) eine Verbindung der Formel IV

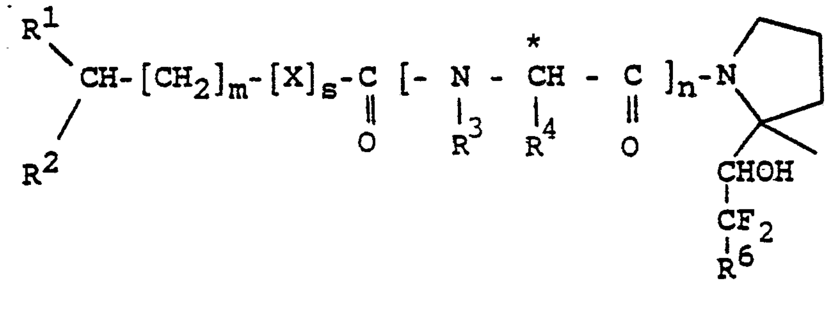

(IV)

in welcher $R^1$, $R^2$, $R^3$, $R^4$, $R^5$, $R^6$, X, m, n und s die gleiche Bedeutung wie in Formel I haben, oxidiert;

b) eine Verbindung der Formel IX

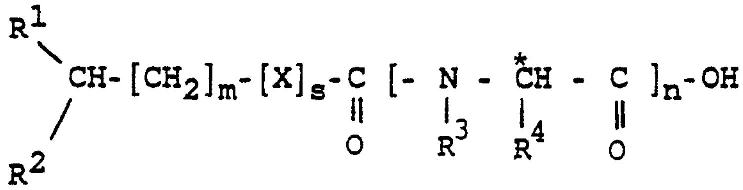

(IX)

in welcher $R^1$, $R^2$, $R^3$, $R^4$, X, m, n und s die gleiche Bedeutung wie in Formel I haben, umsetzt mit einer Verbindung der Formel X

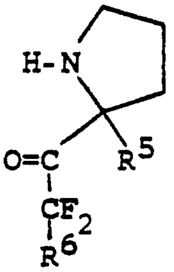

(X)

in welcher $R^5$ und $R^6$ die gleiche Bedeutung wie in Formel I haben, und die nach (a) oder (b) erhaltenen Verbindungen gegebenenfalls in ihre physiologisch verträglichen Salze überführt.

Für Verfahrensvariante (a) kommen folgende Oxidationsmittel in Betracht: Mangandioxid, Natrium-oder Kaliumdichromat, Jones Reagenz ($CrO_3$ in wäßriger Schwefelsäure), N-Bromacetamid, N-Bromsuccinimid, Dimethylsulfoxid, Cerammonnitrat, $CrO_3$ in Pyridin, tert.-Butylchromat, Dipyridin-$CrO_3$, Kaliumhypochlorit oder Jodosobenzol. Als Reaktionsmedium sind Petrolether, Benzol, Tetrachlorkohlenstoff oder, im Falle von $MnO_2$, verdünnte Schwefelsäure geeignet. Die Oxidation wird zwischen 0 °C und dem Siedepunkt des Reaktionsgemisches durchgeführt. Bevorzugt ist als Oxidationsmittel Dimethylsulfoxid mit verschiedenen Zusätzen, wie sie beispielsweise in Houben-Weyl, Band E3, Seiten 275-281 beschrieben wird. Insbesondere bevorzugt sind die Dimethylsulfoxid-Oxidation in Gegenwart von Oxalylchlorid sowie das in J. Org. Chem. 48 [1983] 4155 beschriebene Verfahren.

Verbindungen der Formel IV stellt man beispielsweise durch Umsetzung einer Verbindung der Formel II

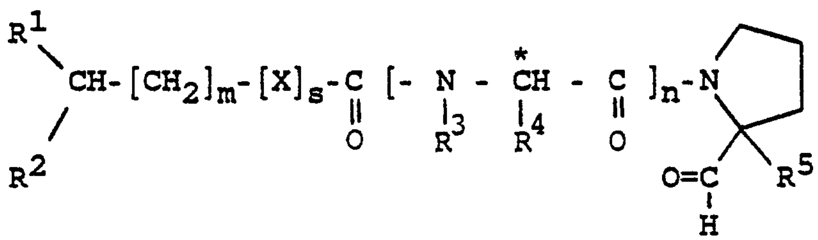

(II)

in welcher $R^1$, $R^2$, $R^3$, $R^4$, $R^5$, X, m, n und s die gleiche Bedeutung wie in Formel I haben, mit einer Verbindung der Formel III

$R^6$-$CF_2$-Y (III)

in welcher $R^6$ die gleiche Bedeutung wie in Formel I hat und Y Halogen, vorzugsweise Chlor, Brom oder Jod bedeutet, in einem inerten Lösungsmittel wie einem Ether, Dimethylformamid unter Zuhilfenahme eines Metalls wie Lithium, Natrium, Kalium, Magnesium oder Zink, wobei das letztere bevorzugt ist, bei 0 °C bis zum Siedepunkt des Lösungsmittels, vorzugsweise bei 20 bis 80 °C mit oder ohne zusätzliche Behandlung mit Ultraschall her.

Die Umsetzung einer Verbindung der Formel IX mit einer Verbindung der Formel X gemäß Verfahrensvariante (b) wird beispielsweise in Analogie zu den in der Peptidchemie gebräuchlichen Amidknüpfungsverfahren, wie sie beispielsweise in Houben-Weyl, Band 15/2, Seite 1-364; in Bodanszky, Bodanszky, "Principles bzw. Practice in Peptide Synthesis", Berlin, 1984 und den US-Patenten 4,331,592 und 4,426,325 beschrieben werden, in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonaten in einem Lösungsmittel wie $CH_3CN$ durchgeführt. Die Verbindungen der Formel II können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76-136). Die Reaktion wird vorzugsweise zwischen -20 °C und dem Siedepunkt des Reaktionsgemisches durchgeführt.

Verbindungen der oben genannten Formel II lassen sich auch erhalten, indem man eine Verbindung der Formel V,

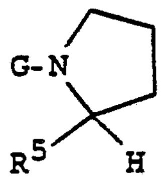

(V)

in der $R^5$ die gleiche Bedeutung wie in Formel I hat und worin G eine Gruppe bedeutet, die die Abspaltung eines Wasserstoffatoms in α-Stellung zum Stickstoffatom erlaubt, insbesondere eine Gruppe der Formel

$R^9$-N=CH-

worin $R^9$ $(C_1-C_8)$-Alkyl, vorzugsweise tert.-Butyl oder $(C_1-C_8)$-Alkoxy-$(C_1-C_8)$-alkyl bedeutet, in einem inerten Lösungsmittel, wie Dialkylether oder Tetrahydrofuran bei -100°C bis 0°C, insbesondere bei -80°C bis -20°C mit einer starken Base, insbesondere einer $(C_1-C_4)$-Alkyllithiumverbindung, Phenyllithium, einem Lithiumdi-$(C_1-C_4)$-alkylamid oder einem Lithium-$(C_5-C_6)$-cycloalkyl-$(C_1-C_4)$-alkylamid und anschließend mit einer Verbindung der Formel VI,

$R^6$-$CF_2$-CHO (VI)

worin $R^6$ die gleiche Bedeutung wie in Formel I hat, umsetzt, wobei man eine Verbindung der Formel VII

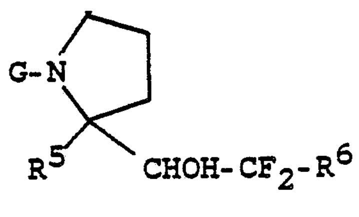

(VII)

in der G, $R^5$ und $R^6$ wie oben definiert sind, erhält, aus dieser die Gruppe G in an sich bekannter Weise, beispielsweise durch Behandeln mit einer Säure oder einer Base, abspaltet und die so erhaltene Verbindung der Formel VIII

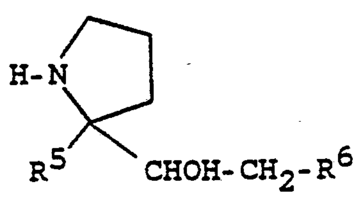

(VIII)

in der $R^5$ und $R^6$ wie oben definiert sind, mit einer Verbindung der oben definierten Formel IX kuppelt. Die Kupplung wird beispielsweise in der bei Verfahrensvariante (a) beschriebenen Weise durchgeführt.

Verbindungen der Formel II sind entweder aus der Literatur bekannt oder lassen sich in Analogie zu literaturbekannten Verfahren herstellen; einige Vertreter sind beispielsweise in den europäischen Patentanmeldungen EP-A-172 458 und EP-A-201 742 und der japanischen Patentanmeldung 1183-297 sowie in Life Sci. 33, 2149 (1983) beschrieben.

Die erfindungsgemäßen Verbindungen der Formel I sind Hemmstoffe der Prolyl-Endopeptidase (EC 3.4.21.26). Von diesem Enzym ist bekannt, daß es Neuropeptide wie Substanz P, Neurotensin, LHRH, TRH, Vasopressin sowie Angiotensin II abbaut (Life Sci. 33, 2149 (1983)). Diese Neuropeptide sind mit wichtigen Funktionen im Zentralnervensystem (ZNS) assoziiert. Durch Hemmung ihres Abbaus mittels Hemmung der Prolyl-Endopeptidase werden durch Verbindungen der Formel I verschiedenartige Wirkungen im ZNS

ausgelöst, insbesondere anti-amnestische, antipsychotische, anxiolytische und antidepressive Wirkungen.

Verbindungen der Formel I eignen sich daher zur Behandlung verschiedener Erkrankungen des Zentralnervensystems, insbesondere als Nootropika und Antipsychotika, bei Warmblütern, vorzugweise beim Menschen. Die Anwendung der erfindungsgemäßen Verbindungen kann intravenös, subcutan oder peroral für sich allein oder in Kombination mit anderen ZNS-wirksamen Substanzen erfolgen.

Die Dosierung liegt je nach Art und Schwere der zu behandelnden Erkrankung bei 0,001-20 mg/kg/Tag, insbesondere bei 0,01-10 mg/kg/Tag. Sie kann in schweren Fällen auch erhöht werden, da toxische Eigenschaften bisher nicht beobachtet wurden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subcutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose-oder Mannitlösungen, oder auch eine Mischung aus den verschiedenen genannten Lösungsmitteln.

**Beispiel 1**

N-(N-Benzyloxycarbonyl-S-prolyl)-2-S-trifluoracetyl-pyrrolidin

a) N-(N-Benzyloxycarbonyl-S-prolyl-2-S-(1-hydroxy-2,2,2-trifluorethyl)-pyrrolidin

In eine Mischung aus 1,65 g N-Benzyloxycarbonyl-S-prolyl-S-prolinal und 0,65 g Zinkpulver in 15 ml Dimethylformamid werden bei 20 °C 10 g Trifluorjodmethan unter Beschallung im Ultraschallbad eingeleitet. Nach 5 Stunden wird mit 100 ml wäßriger 0,1 N Salzsäure versetzt, mit Essigester extrahiert, die vereinigten Essigesterphasen dreimal mit Wasser gewaschen, mit $MgSO_4$ getrocknet und eingeengt. Chromatographie an Kieselgel (Essigester/Cyclohexan (4:1) ergibt 0,6 g der Titelverbindungen als Öl.

$^1$H-NMR ($CDCl_3$):

$\delta$ = 7,4-7,3 (m, 5H); 5,5-4,9 (m, 2H);
4,7-4,4 (m, 1H); 4,3-4,1 (m, 1H);
4,0-3,2 (m, 5H); 2,4-1,8 (m, 8H) ppm.

b) N-(N-Benzyloxycarbonyl-S-prolyl)-2-S-trifluoracetyl-pyrrolidin

0,25 ml Dimethylsulfoxid werden bei -78 °C zu einer Lösung von 0,15 ml Oxalylchlorid in 20 ml Dichlormethan gegeben. Nach 20 Minuten gibt man 0,6 g der Verbindung aus Beispiel 1a) zu, rührt 15 Minuten und gibt dann 1,05 ml Triethylamin zu. Nach weiteren 5 Minuten wird auf Raumtemperatur aufgewärmt, nacheinander mit Wasser, wäßriger 0,1 N Salzsäure, wäßriger 10 %iger $Na_2CO_3$-Lösung und Wasser gewaschen, mit $Na_2SO_4$ getrocknet und eingeengt. Chromatogrphie an Kieselgel ergibt 0,395 g der Titelverbindung als Öl.

$^1$H-NMR ($CDCl_3$):
δ = 7,4-7,2 (m, 5H); 5,25-4,9 (m, 3H);
4,7-4,1 (m, 2H); 3,8-3,4 (m, 3H);
2,4-1,5 (m, 8H) ppm.

**Beispiel 2**

N-[4-(4-Methoxyphenyl)-butyryl]-2-S-trifluoracetyl-pyrrolidin

a) N-[4-(4-Methoxyphenyl)-butyryl]-2-S-(1-hydroxy-2,2,2-trifluorethyl)-pyrrolidin

In eine Mischung aus 1,38 g N-[4-(4-Methoxyphenyl)-butyryl]-S-prolinal und 0,65 g Zinkpulver in 15 ml DMF werden 6 g Trifluorjodmethan bei 20 °C unter Beschallung mit Ultraschall eingeleitet. Nach 1 Stunde wird wie in Beispiel 1a) beschrieben aufgearbeitet. Chromatographie an Kieselgel mit Essigester/Cyclohexan (2:1) ergibt 2 Isomere der Titelverbindung im Verhältnis 2,5:1.

Isomer 1:
$^1$H-NMR ($CDCl_3$):
δ = 7,2-6,6 (m, 4H);
5,5-4,9 (m, 1H);
5,0-4,0 (m, 1H);
3,8 (s, 3H);
3,6-1,6 (m, 10H)ppm.

Isomer 2:
$^1$H-NMR ($CDCl_3$):
δ = 7,2-6,6 (m, 4H);
6,0-5,5 (m, 1H);
4,6-4,3 (m, 1,H);
3,8 (s, 3H);
4,0-3,2 (m, 2H);
3,0-1,7 (m, 10H) ppm.

b) N-[4-(4-Methoxyphenyl)-butyryl)]-2-S-trifluoracetyl-pyrrolidin

Analog dem in Beispiel 1b) beschriebenen Verfahren erhält man aus 160 mg der Verbindung aus Beispiel 2a) 110 mg der Titelverbindung als Öl.
$^1$H-NMR ($CDCl_3$):
δ = 7,2-6,6 (m, 4H); 5,3-4,9 (m, 2H);
4,7-4,2 (m, 1H); 3,8 (s, 3H);
3,6-1,6 (m, 10H) ppm.

## Ansprüche

1. Verbindung der Formel 1

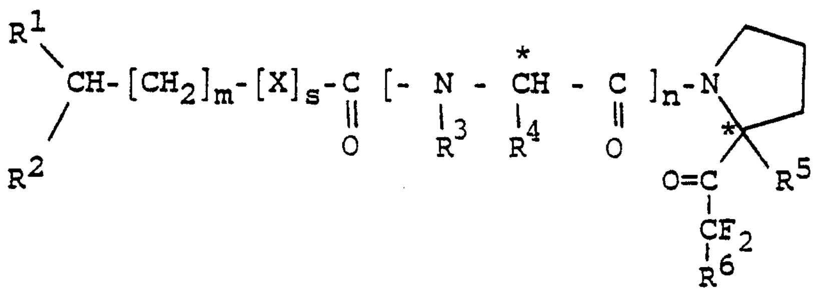

(I)

in welcher

$R^1$ Wasserstoff; $(C_1-C_6)$-Alkyl oder $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino oder ein ($C_1$ oder $C_2$)-Alkylendioxy substituiert ist, bedeutet;

$R^2$ Wasserstoff; $(C_1-C_6)$-Alkyl; $(C_6-C_{12})$-Aryl; $(C_6-C_{12})$-Aryloxy; $(C_7-C_{13})$-Aroyl, Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist;

oder

$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist;

$R^3$ Wasserstoff; $(C_1-C_6)$-Alkyl; $(C_6-C_{12})$-Aryl-$(C_1-C_6)$-alkyl; $(C_5-C_9)$-Cycloalkyl; Indanyl oder Tetrahydronaphthyl bedeutet;

$R^4$ Wasserstoff; $(C_1-C_6)$-Alkyl, das durch Amino, $(C_1-C_6)$-Acylamino oder Benzoylamino monosubstituiert sein kann; $(C_2-C_6)$-Alkenyl; $(C_5-C_9)$-Cycloalkyl; $(C_5-C_9)$-Cycloalkenyl; $(C_5-C_7)$-Cycloalkyl-$(C_1-C_4)$-alkyl; $(C_6-C_{12})$-Aryl oder teilhydriertes $(C_6-C_{12})$-Aryl, die jeweils durch einen, zwei oder drei gleiche oder verschiedene Reste aus der Reihe $(C_1-C_4)$-Alkyl, ($C_1$ oder $C_2$)-Alkoxy und Halogen substituiert sein können; $(C_6-C_{12})$-Aryl-$(C_1-C_4)$-alkyl oder $(C_7-C_{13})$-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen; oder, falls von vorstehenden Definitionen noch nicht umfaßt, die gegebenenfalls geschützte Seitenkette einer natürlich vorkommenden α-Aminosäure bedeutet, oder

$R^3$ und $R^4$ zusammen für -$[CH_2]_p$-stehen, worin p = 3, 4 oder 5 ist und worin eine Methylengruppe durch S oder O ersetzt werden kann;

$R^5$ Wasserstoff; $(C_1-C_6)$-Alkyl; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^6$ $(C_1-C_8)$-Alkyl; $(C_4-C_{10})$-Cycloalkyl oder $(C_4-C_{10})$-Cycloalkyl-$(C_1-C_4)$-alkyl, wobei ein Cycloalkylrest auch, abhängig von der Ringgröße, bis zu 4 Doppelbindungen enthalten und/oder bis zu dreifach verbrückt sein kann; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist; Fluor oder einen Rest $C_qH_{(2q+1-r)}F_r$ mit q = 1, 2, 3, 4 oder 5 und r = eine ganze Zahl von 1 bis (2q+1), bedeutet;

X Sauerstoff; Imino oder N-$(C_1-C_8)$-Alkylimino bedeutet;

m 0, 1, 2, 3, 4 oder 5 ist;

n 0, 1 oder 2 ist und

s 0 oder 1 ist,

sowie deren physiologisch verträgliche Salze, falls solche gebildet werden können.

2. Verbindung der Formel I gemäß Anspruch 1, in welcher
$R^1$ Wasserstoff; ($C_1$-$C_4$)-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;
$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder ein Methylendioxy substituiert sind; oder
$R^1$ und $R^2$ zusammen für Benzyliden stehen, worin der Phenylrest gegebenenfalls durch einen oder zwei gleiche oder verschiedenen Reste aus der Reihe Methyl, Ethyl, Methoxy, Fluor, Chlor, Brom und Hydroxy, drei Methoxy, oder ein Methylendioxy substituiert ist;
$R^3$ Wasserstoff; ($C_1$-$C_4$)-Alkyl; Benzyl; Phenethyl; Cyclopentyl; Cyclohexyl oder Indanyl bedeutet;
$R^4$ Wasserstoff oder die gegebenenfalls geschützte Seitenkette einer natürlich vorkommenden α-Aminosäure bedeutet; oder
$R^3$ und $R^4$ zusammen für einen Rest $-[CH_2]_p$-stehen, der wie im Anspruch 1 definiert ist und worin p = 3 oder 4 ist;
$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;
$R^6$ ($C_1$-$C_8$)-Alkyl; ($C_5$-$C_8$)-Cycloalkyl; Phenyl; Benzyl; Phenethyl; Fluor oder Perfluor-($C_1$-$C_5$)-alkyl bedeutet, wobei Phenyl, Benzyl und Phenethyl im Phenylrest durch einen, zwei oder drei gleiche oder verschiedene Reste aus der Reihe Methyl, Ethyl, Methoxy, Ethoxy, Fluor, Chlor und Brom, oder ein Methylendioxy substituiert sein können;
X Sauerstoff bedeutet;
m = 0, 1, 2, 3, 4 oder 5 ist;
n = 0 oder 1 ist und
s = 0 oder 1 ist,
sowie deren physiologisch verträgliche Salze.

3. Verbindung der Formel 1 gemäß Anspruch 1 oder 2, in welcher
$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 1,3-, 2,4-, 2,5-, 2,6-, 3,4-oder 3,5-Dimethoxyphenyl bedeutet;
$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder
$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-oder 3,5-Dimethoxybenzyliden stehen;
$R^3$ Wasserstoff, Methyl oder Benzyl bedeutet;
$R^4$ Wasserstoff; Methyl; n-Propyl; Isopropyl; n-Butyl; Isobutyl; sec.-Butyl oder Benzyl bedeutet; oder
$R^3$ und $R^4$ zusammen für $-[CH_2]_3$-stehen;
$R^5$ Wasserstoff oder Methyl bedeutet;
$R^6$ ($C_1$-$C_6$)-Alkyl; ($C_5$-$C_8$)-Cycloalkyl; Phenyl; Benzyl; Phenethyl; Fluor oder Perfluor-($C_1$-$C_5$)-alkyl bedeutet, wobei Phenyl, Benzyl und Phenethyl im Phenylrest durch einen oder zwei gleiche oder verschiedene Reste aus der Reihe Methyl, Methoxy, Fluor und Chlor, drei Methoxy, oder ein Methylendioxy substituiert sein können;
X Sauerstoff bedeutet;
m = 0, 1, 2, 3, 4 oder 5 ist;
n = 0 oder 1 ist und
s = 0 oder 1 ist,
sowie deren physiologisch verträgliche Salze.

4. Verbindung der Formel I gemäß einem der Ansprüche 1 bis 3, in welcher
$R^2$ Wasserstoff bedeutet;
$R^3$ und $R^4$ zusammen für $-[CH_2]_3$-stehen;
$R^5$ Wasserstoff bedeutet;
X Sauerstoff bedeutet;

m = 1, 2, 3, oder 4 ist;
n = 1 ist und
s = 0 oder 1 ist,
sowie deren physiologisch verträgliche Salze.

5. Verbindung der Formel I gemäß einem der Ansprüche 1 bis 3, in welcher
$R^5$ Wasserstoff bedeutet;
X Sauerstoff bedeutet;
m = 1, 2, 3 oder 4 ist;
n = 0 ist und
s = 0 oder 1 ist,
sowie deren physiologisch verträgliche Salze.

6. Verfahren zur Herstellung einer Verbindung der Formel I gemäß einem der Ansprüche 1 bis 5, dadurch gekennzeichnet, daß man

a) eine Verbindung der Formel IV

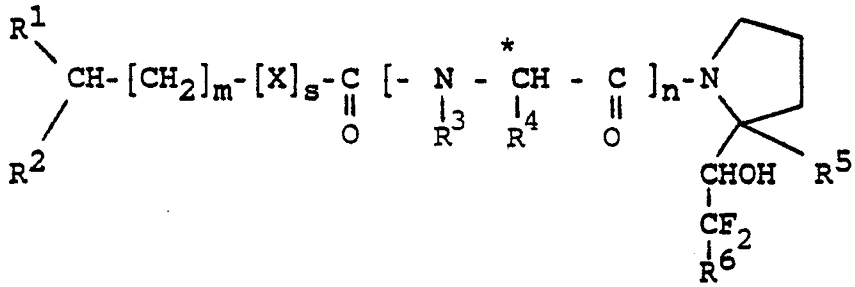

(IV)

in welcher $R^1$, $R^2$, $R^3$, $R^4$, $R^5$, $R^6$, X, m, n und s die gleiche Bedeutung wie in Formel I haben, oxidiert;

b) eine Verbindung der Formel IX

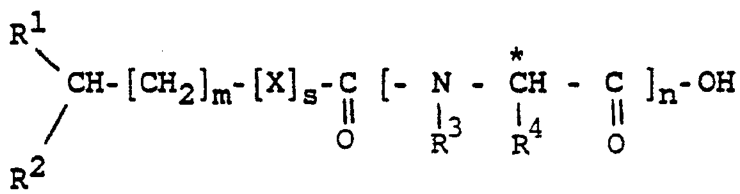

(IX)

in welcher $R^1$, $R^2$, $R^3$, $R^4$, X, m, n and s die gleiche Bedeutung wie in Formel I haben, umsetzt mit einer Verbindung der Formel X

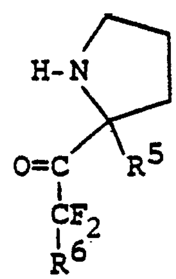

(X)

in welcher $R^5$ und $R^6$ die gleiche Bedeutung wie in Formel I haben, und die nach (a) oder (b) erhaltenen Verbindungen gegebenenfalls in ihre physiologisch verträglichen Salze überführt.

7. Verwendung einer Verbindung gemäß einem der Ansprüche 1 bis 5 als Heilmittel.

8. Verwendung einer Verbindung gemäß einem der Ansprüche 1 bis 5 als Prolyl-Endopeptidase-Hemmer.

9. Pharmazeutische Zubereitung, enthaltend eine Verbindung gemäß einem der Ansprüche 1 bis 5.

10. Verfahren zur Herstellung einer Zubereitung gemäß Anspruch 9, dadurch gekennzeichnet, daß man den Wirkstoff zusammen mit einem physiologisch annehmbaren Träger und gegebenenfalls weiteren Zusatz-, Hilfs-und/oder Konservierungsstoffen in eine geeignete Darreichungsform bringt.

Patentansprüche für die folgenden Vertragsstaaten: ES, GR

1. Verfahren zur Herstellung einer Verbindung der Formel I

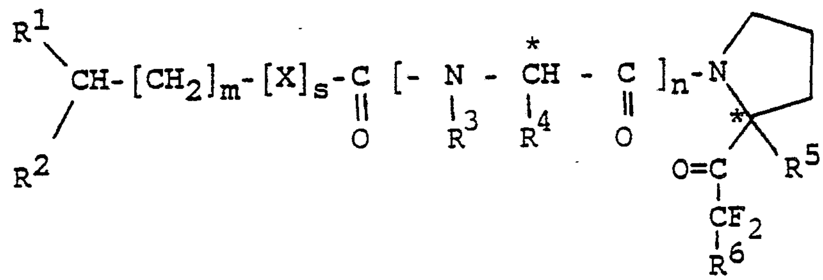

(I)

in welcher

$R^1$ Wasserstoff; ($C_1$-$C_6$)-Alkyl oder ($C_6$-$C_{12}$)-Aryl, das gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino oder ein ($C_1$ oder $C_2$)-Alkylendioxy substituiert ist, bedeutet;

$R^2$ Wasserstoff; ($C_1$-$C_6$)-Alkyl; ($C_6$-$C_{12}$)-Aryl; ($C_6$-$C_{12}$)-Aryloxy; ($C_7$-$C_{13}$)-Aroyl; Hydroxy oder ($C_1$-$C_4$)-Alkoxy bedeutet, wobei Aryl, Aryloxy und Aroyl jeweils gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist;

oder

$R^1$ und $R^2$ zusammen für Benzyliden stehen, worin der Phenylring gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist;

$R^3$ Wasserstoff; ($C_1$-$C_6$)-Alkyl; ($C_6$-$C_{12}$)-Aryl-($C_1$-$C_6$)-alkyl; ($C_5$-$C_9$)-Cycloalkyl; Indanyl oder Tetrahydronaphthyl bedeutet;

$R^4$ Wasserstoff; ($C_1$-$C_6$)-Alkyl, das durch Amino, ($C_1$-$C_6$)-Acylamino oder Benzoylamino monosubstituiert sein kann; ($C_2$-$C_6$)-Alkenyl; ($C_5$-$C_9$)-Cycloalkyl; ($C_5$-$C_9$)-Cycloalkenyl; ($C_5$-$C_7$)-Cycloalkyl-($C_1$-$C_4$)-alkyl; ($C_6$-$C_{12}$)-Aryl oder teilhydriertes ($C_6$-$C_{12}$)-Aryl, die jeweils durch einen, zwei oder drei gleiche oder verschiedene Reste aus der Reihe ($C_1$-$C_4$)-Alkyl, ($C_1$ oder $C_2$)-Alkoxy und Halogen substituiert sein können; ($C_6$-$C_{12}$)-Aryl-($C_1$-$C_4$)-alkyl oder ($C_7$-$C_{13}$)-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen; oder, falls von vorstehenden Definitionen noch nicht umfaßt, die gegebenenfalls geschützte Seitenkette einer natürlich vorkommenden α-Aminosäure bedeutet, oder

$R^3$ und $R^4$ zusammen für -$[CH_2]_p$-stehen, worin p = 3, 4 oder 5 ist und worin eine Methylengruppe durch S oder O ersetzt werden kann;

$R^5$ Wasserstoff; ($C_1$-$C_6$)-Alkyl; ($C_6$-$C_{12}$)-Aryl-($C_1$-$C_5$)-alkyl, das im Arylteil gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist, bedeutet;

$R^6$ ($C_1$-$C_8$)-Alkyl; ($C_4$-$C_{10}$)-Cycloalkyl oder ($C_1$-$C_{10}$)-Cycloalkyl-($C_1$-$C_4$)-alkyl, wobei ein Cycloalkylrest auch, abhängig von der Ringgröße, bis zu 4 Doppelbindungen enthalten und/oder bis zu dreifach verbrückt sein kann; ($C_6$-$C_{12}$)-Aryl-($C_1$-$C_5$)-alkyl, das im Arylteil gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist; Fluor oder einen Rest $C_qH_{(2q+1-r)}F_r$ mit q = 1, 2, 3, 4 oder 5 und r = eine ganze Zahl von 1 bis (2q+1), bedeutet;

X Sauerstoff; Imino oder N-($C_1$-$C_8$)-Alkylimino bedeutet;

m 0, 1, 2, 3, 4 oder 5 ist;

n 0, 1 oder 2 ist und

s 0 oder 1 ist,

sowie deren physiologisch verträgliche Salze, falls solche gebildet werden können, dadurch gekennzeichnet, daß man

a) eine Verbindung der Formel IV

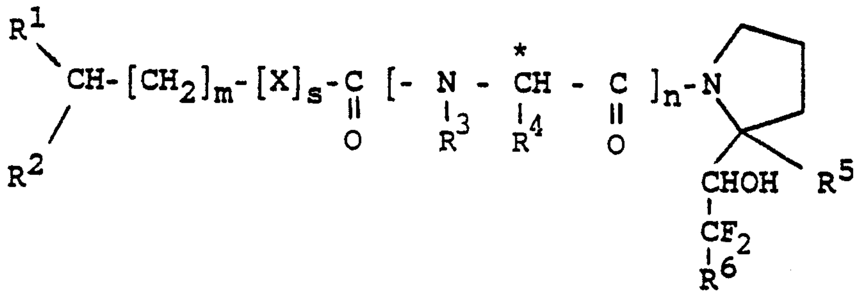

(IV)

in welcher $R^1$, $R^2$, $R^3$, $R^4$, $R^5$, $R^6$, X, m, n und s die gleiche Bedeutung wie in Formel I haben, oxidiert;

b) eine Verbindung der Formel IX

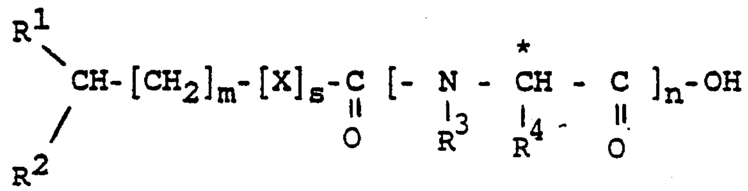

(IX)

in welcher $R^1$, $R^2$, $R^3$, $R^4$, X, m, n und s die gleiche Bedeutung wie in Formel I haben, umsetzt mit einer Verbindung der Formel X

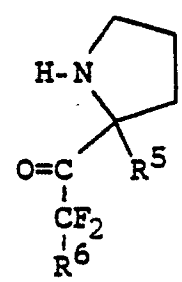

(X)

in welcher $R^5$ und $R^6$ die gleiche Bedeutung wie in Formel I haben, und die nach (a) oder (b) erhaltenen Verbindungen gegebenenfalls in ihre physiologisch verträglichen Salze überführt.

2. Verfahren gemäß Anspruch 1, dadurch gekennzeichnet, daß man eine Verbindung der Formel I herstellt, in welcher

$R^1$ Wasserstoff; $(C_1\text{-}C_4)$-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder ein Methylendioxy substituiert sind; oder

$R^1$ und $R^2$ zusammen für Benzyliden stehen, worin der Phenylrest gegebenenfalls durch einen oder zwei gleiche oder verschiedenen Reste aus der Reihe Methyl, Ethyl, Methoxy, Fluor, Chlor, Brom und Hydroxy, drei Methoxy, oder ein Methylendioxy substituiert ist;

$R^3$ Wasserstoff; $(C_1\text{-}C_4)$-Alkyl; Benzyl; Phenethyl; Cyclopentyl; Cyclohexyl oder Indanyl bedeutet;

$R^4$ Wasserstoff oder die gegebenenfalls geschützte Seitenkette einer natürlich vorkommenden α-Aminosäure bedeutet; oder

$R^3$ und $R^4$ zusammen für einen Rest $-[CH_2]_p$-stehen, der wie im Anspruch 1 definiert ist und worin p = 3 oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ $(C_1\text{-}C_8)$-Alkyl; $(C_5\text{-}C_8)$-Cycloalkyl; Phenyl; Benzyl; Phenethyl; Fluor oder Perfluor-$(C_1\text{-}C_5)$-alkyl bedeutet, wobei Phenyl, Benzyl und Phenethyl im Phenylrest durch einen, zwei oder drei gleiche oder verschiedene

Reste aus der Reihe Methyl, Ethyl, Methoxy, Ethoxy, Fluor, Chlor und Brom, oder ein Methylendioxy substituiert sein können;
X Sauerstoff bedeutet;
m = 0, 1, 2, 3, 4 oder 5 ist;
n = 0 oder 1 ist und
s = 0 oder 1 ist,
sowie deren physiologisch verträgliche Salze.

3. Verfahren gemäß Anspruch 1 oder 2, dadurch gekennzeichnet, daß man eine Verbindung der Formel I herstellt, in welcher
$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 1,3-, 2,4-, 2,5-, 2,6-, 3,4-oder 3,5-Dimethoxyphenyl bedeutet;
$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder
$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-oder 3,5-Dimethoxybenzyliden stehen;
$R^3$ Wasserstoff, Methyl oder Benzyl bedeutet;
$R^4$ Wasserstoff, Methyl; n-Propyl; Isopropyl; n-Butyl; Isobutyl; sec.-Butyl oder Benzyl bedeutet; oder
$R^3$ und $R^4$ zusammen für $-[CH_2]_3$-stehen;
$R^5$ Wasserstoff oder Methyl bedeutet;
$R^6$ $(C_1-C_6)$-Alkyl; $(C_5-C_8)$-Cycloalkyl; Phenyl; Benzyl; Phenethyl; Fluor oder Perfluor-$(C_1-C_5)$-alkyl bedeutet, wobei Phenyl, Benzyl und Phenethyl im Phenylrest durch einen oder zwei gleiche oder verschiedene Reste aus der Reihe Methyl, Methoxy, Fluor und Chlor, drei Methoxy, oder ein Methylendioxy substituiert sein können;
X Sauerstoff bedeutet;
m = 0, 1, 2, 3, 4 oder 5 ist;
n = 0 oder 1 ist und
s = 0 oder 1 ist,
sowie deren physiologisch verträgliche Salze.

4. Verfahren gemäß einem der Ansprüche 1 bis 3, dadurch gekennzeichnet, daß man eine Verbindung der Formel I herstellt, in welcher
$R^2$ Wasserstoff bedeutet;
$R^3$ und $R^4$ zusammen für $-[CH_2]_3$-stehen;
$R^5$ Wasserstoff bedeutet;
X Sauerstoff bedeutet;
m = 1, 2, 3 oder 4 ist;
n = 1 ist und
s = 0 oder 1 ist,
sowie deren physiologisch verträgliche Salze.

5. Verfahren gemäß einem der Ansprüche 1 bis 3, dadurch gekennzeichnet, daß man eine Verbindung der Formel I herstellt, in welcher
$R^5$ Wasserstoff bedeutet;
X Sauerstoff bedeutet;
m = 1, 2, 3 oder 4 ist;
n = 0 ist und
s = 0 oder 1 ist,
sowie deren physiologisch verträgliche Salze.

6. Verfahren zur Herstellung einer Zubereitung enthaltend eine Verbindung gemäß einem der Ansprüche 1 bis 5, dadurch gekennzeichnet, daß man den Wirkstoff zusammen mit einem physiologisch annehmbaren Träger und gegebenenfalls weiteren Zusatz-, Hilfs-und/oder Konservierungsstoffen in eine geeignete Darreichungsform bringt.